# EUROPEAN PATENT APPLICATION

(11) **EP 1 238 650 A2**
(43) Date of publication of application: **11.09.2002**
(21) Application number: 02290576.4
(22) Date of filing: 07.03.2002
(51) Int. Cl.: A61K 7/16

(54) **Antimicrobial flavor and oral care composition containing the same**

(30) Priority: 07.03.2001 JP 2001062916
(71) Applicant: Takasago International Corporation, Tokyo 144-8721 (JP)
(72) Inventor: Hanada, Minoru, Takasago International Corporation, Hiratsuka-shi, Kanagawa 254-0073 (JP); Arai, Haruo, Takasago International Corporation, Hiratsuka-shi, Kanagawa 254-0073 (JP)
(74) Representative: Uchida, Kenji

(57) **Abstract**

The present invention provides antimicrobial flavors and flavor compositions which are of high safety and little influential on intestinal bacteria, as well as oral care compositions and foods containing the same. The flavor is selected from dihydrofarnesol, isocamphylcyclohexanol, levosandol, bacdanol, Santalinol, methyl atrarate, 10-undecen-1-ol, 2-methyl undecanal, sandalol, dodecanal, brahmanol, neroridol, o-methoxycinnamic aldehyde, methyl sandeflor, 9-decen-1-ol, 3-dodecenal, etc.

## Description

### FIELD OF THE INVENTION

The present invention relates to antimicrobial flavors which are of high safety and effectively inhibitory on pathogenic oral bacterial involved in periodontal disease (paradentosis) or dental caries. The present invention also relates to oral care compositions and foods which contain the antimicrobial flavors.

Several hundreds of kinds of microorganisms such as bacteria and fungi inhabit the mouth and are involved in oral diseases. Proliferation of pathogens out of these oral microorganisms is induced by some factors and causes various oral diseases such as dental caries, paradentosis, halitosis or bad breath. In particular, caries and paradentosis are the two most common oral diseases, and prevention and treatment thereof are a matter of great concern. Pathogenic bacteria known to be causative of caries include *Streptococcus mutans*, *Actinomyces naeslundii*, and *Actinomyces viscosus.* Those causing paradentosis include *Fusobacterium nucleatum, porphyromonas gingivalis*, and *Prevotella intermedia.*

Synthetic antimicrobials or antibiotics have hitherto been used in an attempt to eliminate these pathogens from the oral cavity, but it has been pointed out that these substances can cause emergence of resistant bacteria, toxicity through long-term use, and destruction of bacterial balance in the intestines due to their strong action. Therefore antimicrobial agents which are of little toxicity and little influential on intestinal bacteria have been desired.

On the other hand, it has been widely known that essential oils or fragrance components thereof have antibacterial and antifungal activities (see Asakoshi Toru, *J. Soc. Sosm. Chem. Japan,* vol. 34, pp. 25-46 (2000) and the references cited therein). It is known that they manifest their effects not only on direct contact with test microorganisms but in a vapor phase. Most of the researches have been confined to activities against aerobic Gram-positive or negative bacteria, yeasts and filamentous fungi. With respect to anaerobes, although there are many reports of activities on *Propionibacterium acnes,* which causes acne, reports on activities against oral bacteria, which are also anaerobes, are few.

Saeki et al. researched inhibitory activities of essential oils and their components on oral bacteria and revealed that hinokitiol, cinnamic aldehyde, thymol, eugenol, etc. are active (*Bull*. *Tokyo Den. Coll*., vol. 30, No. 3, pp. 129-135 (1989)). JP-B-62-58327 (1987) describes activities of spice extracts such as all spice on *Fusobacterium nucleatum*, and JP-B-4-32047 (1992) teaches that a composition comprising hinokitiol, a flavor selected from cresyl acetate, cyclamenaldehyde, isoeugenol, heliotropin, n-decanal, citral, δ-dodecalactone, and the like, and a carboxylic acid, etc. is inhibitory on *Streptococcus mutans*. Activities on other bacteria are not described.

### SUMMARY OF THE INVENTION

An object of the present invention is to provide an antimicrobial agent which is of little toxicity, little influential on intestinal bacteria and useful as an active ingredient of an oral care composition.

Another object of the invention is to provide oral care compositions and foods containing the antimicrobial agent.

The present inventors have intensively studied activities of flavors on pathogenic oral bacteria causing paradentosis or dental caries and found as a result that some of flavors exhibit powerful inhibitory activities. The present invention has been completed based on this finding.

The present invention provides
1. an antimicrobial flavor to be added to an oral care composition which is selected from the group consisting of dihydrofarnesol, isocamphylcyclohexanol, levosandol, bacdanol, Santalinol, methyl atrarate, 10-undecen-1-ol, 2-methyl undecanal, sandalol, dodecanal, brahmanol, neroridol, o-methoxycinnamic aldehyde, methyl sandeflor, 9-decen-1-ol, 3-dodecenal, acetyl cedrene, nootkatone, cinnamic aldehyde dimethyl acetal (DMA), lilial, 2-methyl decanal, perilla alcohol, 1-nonanol, undecanal, trans-2-undecenal, dihydroeugenol, helional, isobutyl quinoline, 3-menthoxypropane-1,2-diol, phenylacetaldehyde, Cashmerane, 5-cyclohexadecen-1-one, ambroxan, cedrol, cedryl acetate, cyclopentadecanone, ethylenedodecanedioate, cyclopentadecanolide, phantolid, and tonalid;
2. an oral care composition containing the antimicrobial flavor of 1. above; and
3. an oral care composition of 2. above, wherein the oral composition is a tooth paste,
4. an oral care composition of 2. above, wherein the oral composition is mouth wash or liquefied tooth paste,
5. a food containing the antimicrobial flavor of 1. above.

### DETAILED DESCRIPTION OF THE INVENTION

The present invention is based on the potent antimicrobial activities found in the above-described flavor components that have high safety as supported by long use against pathogenic oral microorganisms and proposes adding these flavor components to an oral care composition or a food to control pathogens causing various oral diseases, such as dental caries, paradentosis, halitosis or bad breath, thereby enabling prevention of these diseases.

Dihydrofarnesol, isocamphylcyclohexanol, levosandol, bacdanol, Santalinol, methyl atrarate, 10-undecen-1-ol, 2-methyl undecanal, sandalore, dodecanal, brahmanol, neroridol, o-methoxycinnamic aldehyde, methyl sandeflor, 9-decen-1-ol, 3-dodecenal, acetyl cedrene, nootkatone, cinnamic aldehyde dimethyl acetal (DMA), lilial, 2-methyl decanal, perilla alcohol, 1-nonanol, undecanal, trans-2-undecenal, dihydroeugenol, helional, isobutyl quinoline, 3-menthoxypropane-1,2-diol, phenylacetaldehyde, Cashmerane, 5-cyclohexadecen-1-one, ambroxan, cedrol, cedryl acetate, cyclopentadecanone, ethylenedodecanedioate, cyclopentadecanolide, phantolid, and tonalid shown in 1. above are flavor components having antimicrobial action on pathogenic oral bacteria, such as caries-causing bacteria and paradentosis-causing bacteria. They have been in long use as fragrances and flavors and are of high safety and little influential on intestinal bacteria. These flavors are commercially available or can be prepared by conventional methods.

The flavors can be used either individually or as a mixture of two or more thereof (flavor composition). When two or more thereof are added jointly, the antimicrobial action can be exhibited in a lower amounts than that required in a single use. Where the antimicrobial flavor is compounded with other flavor base (so-called body base), it is advantageous in reducing the influences on the body base. Further, two or more flavors can be compounded into a flavor composition with a desired aroma different from each of them. When two or more are used in combination, the compounding ratio is not particularly limited.

The antimicrobial flavors of the invention can be mixed into an oral care composition or a food either individually or as an antimicrobial flavor composition which combines the antimicrobial compound alone and/or as an antimicrobial flavor composition combined with other flavor base (body base). Where the antimicrobial flavor or flavors is/are mixed with a body base, the mixing ratio of the antimicrobial flavor to the body base is not particularly limited. The mixing ratio of the antimicrobial flavor(s) to an oral care composition is not limited, either. A preferable ratio of the antimicrobial flavor(s) in the resulting oral care composition is usually 0.005 to 10% (given by weight, hereinafter the same), more preferably 0.02 to 3%. A ratio less than 0.005% may sometimes fails to manifest a sufficient antimicrobial effect. A ratio exceeding 10% may sometimes spoil the flavor of the oral care composition.

The oral care compositions intended in the present invention include tooth paste, mouth wash, and liquefied tooth paste. These oral care compositions can contain appropriate components other than the above-described antimicrobial flavor (or flavor composition) in accordance with the purpose, the kind and the like of the composition.

For example, tooth paste can comprise abrasives, such as calcium secondary phosphate dihydrate, calcium carbonate, and silicic anhydride; surface active agents, such as sodium laurylsulfate and polyoxyethylene alkyl sulfates; foaming agents, such as sucrose fatty acid esters, maltose fatty acid esters, and sodium lauroyl sarcosinate; wetting agents, such as glycerol, sorbitol, and propylene glycol; and thickeners, such as carboxymethyl cellulose, sodium alginate, carrageenan, and xanthan gum.

Tooth paste can further contain antiinflammatory agents, such as tranexamic acid, dipotassium glycyrrhetinate, and dl-α-tocopheryl acetate; astringents, such as aluminum lactate; sweeteners, such as sodium saccharin, stevioside, glycyrrhizin, and xylitol; natural flavor sources, such as peppermint oil, spearmint oil, lemon oil, and sage oil; flavors, such as menthol, eugenol, and limonene; and preservatives, such as benzoic acid derivatives, e.g., sodium benzoate, sodium p-hydroxybenzoate, and butyl p-hydroxybenzoate, and sodium salicylate.

Tooth paste may also contain, as an active ingredient in addition to the antimicrobial flavor of the invention, cationic bactericides, such as chlorhexidine and benzalkonium chloride; phenolic compounds, such as triclosan and hinokitiol; enzymes, such as dextranase, lysozyme, lytic enzymes, and superoxide desmutase; and fluorides, such as sodium fluoride.

Mouth wash and liquefied tooth paste can be prepared by compounding the antimicrobial flavor or a flavor composition containing the same with the above-described various other ingredients such as surface active agents, flavors, and sweeteners.

The foods to which the present invention is suitably applied include those which stay in the mouth for a given time, such as chewing gum, candy, troches, gummy jelly, and lozenge. The antimicrobial flavor of the invention can be incorporated into these foods in a concentration of 0.005 to 5%, preferably 0.02 to 2%.

The present invention also provides a method of preventing periodontal disease (paradentosis) or dental caries which comprises applying the antimicrobial flavor or the antimicrobial flavor composition described above to the oral cavity. The method for administration is not particularly limited. For example, the antimicrobial flavor or the antimicrobial flavor composition can be suitably applied by using oral care composition described above (tooth paste, mouth wash or liquefied tooth paste) or by eating the foods described above. In such a case, the oral care composition or food may preferably used once or more per day, and preferably stayed in the oral cavity for 1 minute or more, more preferably 2 minutes or more, and most preferably 5 minutes or more. The present invention can also relate to use of the antimicrobial flavor or the antimicrobial flavor composition described above for the manufacture of the oral care composition or food for preventing periodontal disease (paradentosis) or dental caries.

The present invention will now be illustrated in greater detail with reference to Examples, but it should be understood that the invention is not limited thereto. Measurement of antimicrobial activity (minimum inhibitory concentration; MIC):

The minimum inhibitory activities of the antimicrobial flavors of the invention on anaerobic oral bacteria were measured by an agar plate dilution method.

All the test strains were obtained from Riken (The Institute of Physical and Chemical Research). The names and abbreviations of the strains are shown in Table 1 below (The abbreviations are used in the following description).

**TABLE 1**

| Abbrev. | Strain Name |
|---|---|
| Su-1 | *Streptococcus mutans* JCM 5175 |
| Aa-1 | *Actinomyces naeslundii* JCM 8350 |
| Av-1 | *Actinomyces viscosus* JCM 8352 |
| Fn-1 | *Fusobacterium nucleatum* JCM 6328 |
| Pg-1 | *Porphyromonas gingivalis* JCM 8525 |
| Pt-1 | *Prevotella intermedia* JCM 6322 |

### EXAMPLE 1

### (1) Preparation of test microbial cell suspension (inoculum)

Each test microorganism kept frozen (-80°C) in glycerol at a concentration of 25% was thawed. A 100 µl portion of the suspension was inoculated into 10 ml of a liquid medium. A Gifu university anaerobic medium (GAM) (Nissui Pharmaceutical Co., Ltd) having added thereto Hemin (Tokyo Kasei) and Menadione (Sigma) was used for Su-1 and Fn-1. Schaedler Broth (BBL) having added thereto Hemin and Menadione was used for the other strains. The culture was anaerobically incubated at 37°C for 72 hours by using BBL Gas Pack Anaerobic System. After the cultivation, the microbial cell suspension of Su-1 was 10-fold diluted to prepare an inoculum. The cultures of Fn-1 and Pg-1 were used as such as an inoculum. The cultures of Pt-1, Aa-1, and Av-1 were subjected to centrifugation, and the separated microbial cells were suspended in 2 ml of sterilized physiological saline to prepare the respective inoculums.

### (2) Measurement of minimum inhibitory concentration

An agar medium prepared by adding 2% agar (Difco), Hemin, and Menadione to Trypticase Soy Broth (BBL) was used. A test sample (antimicrobial flavor) was dissolved in ethanol to prepare 2-fold serial dilutions. To 10 ml of the agar medium was added 100 µl of each dilution and stirred well. The agar medium was transferred to a petri dish (diameter: 9 cm) and solidified at room temperature. The solidified agar medium was inoculated with 5 µl of the inoculum by means of a multipoint inoculator (Microplanter MIT-P27, supplied by Sakuma Seisakusho), followed by culturing at 37°C for 72 hours under anaerobic conditions. The growth of the test microorganism was compared with a blank petri dish (the agar medium to which 100 µl of ethanol had been added). The lowest sample concentration that inhibited growth was taken as MIC (ppm). The results obtained are shown in Table 2 and Table 3.

**TABLE 2**

| Antimicrobial Flavor | Caries-Related Bacteria | | | Paradentosis-Related Bacterial | | |
|---|---|---|---|---|---|---|
| | Su-1 | Aa-1 | Av-1 | Fn-1 | Fg-1 | Pt-1 |
| Dihydrofarnesol | 31.3 | 31.3 | 62.5 | 31.3 | 7.8 | 7.8 |
| Isocamphylcyclohexanol | 31.3 | 31.3 | 31.3 | 31.3 | 3.9 | 3.9 |
| Levosandol | 125 | 62.5 | 62.5 | 62.5 | 31.3 | 31.3 |
| Bacdanol | 125 | 125 | 62.5 | 62.5 | 31.3 | NG |
| Santalinol | 250 | 62.5 | 62.5 | 125 | 15.6 | 31.3 |
| Methyl atrarate | >1000 | 62.5 | 62.5 | 125 | 31.3 | 62.5 |
| 10-undecen-1-ol | 250 | 125 | 125 | 125 | 31.3 | 31.3 |
| 2-Methyl undecanal | 250 | 125 | 125 | 125 | 62.5 | 62.5 |
| Sandalore | 250 | 125 | 125 | 125 | 31.3 | 31.3 |
| Dodecanal | 500 | 500 | 500 | 125 | 62.5 | 62.5 |
| Brahmanol | 250 | 125 | 125 | 250 | 31.3 | 31.3 |
| Neroridol | 500 | 125 | 125 | 50 | 31.3 | 31.3 |
| o-Methoxycinnamic aldehyde | 250 | 250 | 250 | 250 | 7.8 | 62.5 |
| Methyl sandeflor | 500 | 250 | 125 | 250 | 62.5 | 62.5 |
| 9-decen-1-ol | 1000 | 250 | 250 | 250 | 125 | 62.5 |
| 3-Dodecenal | 1000 | 500 | 500 | 250 | 62.5 | 31.3 |

**TABLE 3**

| Antimicrobial Flavor | Caries-Related Bacteria | | | Paradentosis-Related Bacterial | | |
|---|---|---|---|---|---|---|
| | Su-1 | Aa-1 | Av-1 | Fn-1 | Fg-1 | Pt-1 |
| Acetyl cedrene | 250 | 31.3 | 31.3 | 500 | 15.6 | NG |
| Nootkatone | 1000 | 62.5 | 62.5 | 500 | 7.8 | NG |
| Cinnamic aldehyde dimethyl acetal | 250 | 125 | 250 | 500 | <15.6 | 62.5 |
| Lilial | 500 | 125 | 500 | 500 | 62.5 | 62.5 |
| 2-Methyl decanal | 500 | 250 | 500 | 500 | 62.5 | 125 |
| Perilla alcohol | 500 | 250 | 500 | 500 | 125 | NG |
| 1-nonanol | 1000 | 500 | 500 | 500 | 250 | 250 |
| Undecanal | 1000 | 500 | 500 | 500 | 125 | NG |
| Trans-2-undecenal | 250 | 125 | 125 | 1000 | NG | NG |
| Dihydroeugenol | 1000 | 500 | 500 | 500 | 62.5 | 62.5 |
| Helional | 1000 | 250 | 500 | 1000 | 125 | 1000 |
| Isobutyl quinoline | 1000 | 250 | 250 | 1000 | 62.5 | 250 |
| 3-Menthoxypropane-1,2-diol | 1000 | 500 | 62.5 | 1000 | 250 | NG |
| Phenylacetaldehyde | >1000 | 500 | 250 | 1000 | 15.6 | 15.6 |
| Cashmerane | >1000 | 1000 | 1000 | >1000 | 250 | 250 |
| 5-cyclohexadecen-1-one | >1000 | >1000 | >1000 | >1000 | 15.6 | 31.3 |
| Ambroxan | >1000 | >1000 | >1000 | >1000 | 31.3 | 31.3 |
| Cedrol | >1000 | >1000 | >1000 | >1000 | 31.3 | 31.3 |
| Cedryl acetate | >1000 | >1000 | >1000 | >1000 | 31.3 | 62.5 |
| Cyclopentadecanone | >1000 | >1000 | >1000 | >1000 | <62.5 | <62.5 |
| Ethylenedodecanedioate | >1000 | >1000 | 250 | >1000 | 31.3 | >1000 |
| Cyclopentadecanolide | >1000 | >1000 | >1000 | >1000 | <62.5 | <62.5 |
| Phantolid | >1000 | >1000 | >1000 | >1000 | 31.3 | 62.5 |
| Tonalid | >1000 | >1000 | >1000 | >1000 | 15.6 | 62.5 |

As demonstrated in Tables 2 and 3, the flavors according to the invention exhibit potent antimicrobial activities on dental caries-causing bacteria and paradentosis-causing bacteria. In Table 2, NG means the test microorganism did not grow to give no results, > indicates that growth was not inhibited at the concentration described, and < indicates that a test was not carried out at or below the concentration described.

### EXAMPLE 2

### (1) Preparation of antimicrobial flavor base

An antimicrobial flavor base (aromahygiene base; hereinafter referred to as AHB) was prepared according to The formulation (wt%) is shown in Table 4.

**TABLE 4**

| Name of Flavor | Amount |
|---|---|
| Dihydrofarnesol | 50 |
| Neroridol | 30 |
| Dodecanal | 5 |
| Nootkatone | 5 |
| 2-Methyl decanal | 5 |
| Trans-2-undecenal | 5 |

### (2) Preparation of antimicrobial flavor composition

The AHB prepared in (1) above was compounded into each of a peppermint body base-1 (hereinafter referred to as ZX-4693), a peppermint body base-2 (hereinafter referred to as ZX-4917), and a spearmint body base (hereinafter referred to as ZX-4980) to prepare an antimicrobial flavor composition. For comparison, the equal amount of ethanol was mixed into each body base. The formulations (wt%) are shown in Table 5 below.

**TABLE 5**

| | ZX-4693 | ZX-4917 | ZX-4980 | AHB | Ethanol |
|---|---|---|---|---|---|
| Example 2-1 | 85 | - | - | 15 | - |
| Compara. Example 2-1 | 85 | - | - | - | 15 |
| Example 2-2 | - | 75 | - | 25 | - |
| Compara. Example 2-2 | - | 75 | - | - | 25 |
| Example 2-3 | - | - | 85 | 15 | - |
| Compara. Example 2-3 | - | - | 85 | - | 15 |

### (3) Antimicrobial activity of aromahygiene base (AHB) and antimicrobial flavor composition

Antimicrobial activities (MIC; ppm) of the aromahygiene base (AHB) prepared in (1) and the antimicrobial flavor compositions prepared in (2) (Examples 2-1 to 2-3) and Comparative Examples 2-1 to 2-3 were measured in the same manner as in Example 1 (2). The results obtained are shown in Table 6.

**TABLE 6**

| | Caries-related Bacteria | | | Paradentosis-related Bacterial | | |
|---|---|---|---|---|---|---|
| | Su-1 | Aa-1 | Av-1 | Fn-1 | Pg-1 | Pt-1 |
| Aromahygiene base (AHB) | 62.5 | 125 | 62.5 | 31.3 | 7.8 | 7.8 |
| Ex. 2-1 | 250 | 250 | 250 | 250 | 31.3 | 31.3 |
| Comp. Ex. 2-1 | >2000 | >2000 | >2000 | 2000 | 1000 | 1000 |
| Ex. 2-2 | 125 | 125 | 125 | 125 | 15.6 | 15.6 |
| Comp. Ex. 2-2 | >2000 | >2000 | >2000 | 2000 | 1000 | 1000 |
| Ex. 2-3 | 250 | 1000 | 250 | 250 | 62.5 | 62.5 |
| Comp. Ex. 2-3 | >2000 | >2000 | >2000 | >2000 | 2000 | 2000 |

As can be seen from Table 6, the aromahygiene base (AHB) prepared in (1) and the antimicrobial flavor compositions containing the aromahygiene base prepared in (2) exhibit potent antimicrobial activities compared with the corresponding Comparative Examples (2-1 to 2-3).

### EXAMPLE 3

Tooth paste was prepared in a conventional manner according to the following formulation.

| Composition | wt% |
|---|---|
| Calcium phosphate | 30 |
| Glycerol | 10 |
| Sorbitol | 20 |
| Sodium laurylsulfate | 1.5 |
| Sodium carboxymethyl cellulose | 1.0 |
| Carrageenan | 0.1 |
| Sodium saccharin | 0.1 |
| Antimicrobial flavor composition of Example 2-1 | 1.0 |
| Sodium benzoate | 0.3 |
| Water | balance |

### EXAMPLE 4

Mouth wash was prepared in a conventional manner according to the following formulation.

| Composition | wt% |
|---|---|
| Ethanol | 10.0 |
| Glycerol | 5.0 |
| Citric acid | 0.01 |
| Sodium citrate | 0.1 |
| Polyoxyethylene hydrogenated castor oil | 0.5 |
| Methyl p-hydroxybenzoate | 0.1 |
| Antimicrobial flavor composition of Example 2-3 | 0.5 |
| Water | balance |

### EXAMPLE 5

Plate-form chewing gum of peppermint flavor was prepared in a conventional manner according to the following formulation.

| Composition | wt% |
|---|---|
| Plate gum base | 24.0 |
| Starch syrup | 13.0 |
| Powder sugar | 62.0 |
| Antimicrobial flavor composition of Example 2-2 | 1.0 |
| Color | q.s. |

### EXAMPLE 6

Candy of spearmint flavor was prepared in a conventional manner according to the following formulation.

| Composition | wt% |
|---|---|
| Granular sugar | 44.5 |
| Starch syrup | 41.0 |
| Water | 14.0 |
| Antimicrobial flavor composition of Example 2-3 | 0.5 |
| Color | q.s. |

The present invention provides antimicrobial flavors and flavor compositions which are of high safety and little influential on intestinal bacteria and exhibit potent inhibitory activities on oral pathogenic microorganisms causing dental caries and paradentosis and are therefore useful as an active ingredient of oral care compositions. The present invention also provides oral care compositions, such as tooth paste and mouth wash, and foods which contain an antimicrobial flavor or flavor composition effectively exhibiting inhibitory activities on the oral pathogenic microorganisms.

While the invention has been described in detail and with reference to specific embodiments thereof, it will be apparent to one skilled in the art that various changes and modifications can be made therein without departing from the scope thereof.

## Claims

1. An antimicrobial flavor to be added to an oral care composition which comprises at least one compound selected from the group consisting of dihydrofarnesol, isocamphylcyclohexanol, levosandol, bacdanol, Santalinol, methyl atrarate, 10-undecen-1-ol, 2-methyl undecanal, sandalore, dodecanal, brahmanol, neroridol, o-methoxycinnamic aldehyde, methyl sandeflor, 9-decen-1-ol, 3-dodecenal, acetyl cedrene, nootkatone, cinnamic aldehyde dimethyl acetal (DMA), lilial, 2-methyl decanal, perilla alcohol, 1-nonanol, undecanal, trans-2-undecenal, dihydroeugenol, helional, isobutyl quinoline, 3-menthoxypropane-1,2-diol, phenylacetaldehyde, Cashmerane, 5-cyclohexadecen-1-one, ambroxan, cedrol, cedryl acetate, cyclopentadecanone, ethylenedodecanedioate, cyclopentadecanolide, phantolid, and tonalid.

2. An oral care composition comprising the antimicrobial flavor according to claim 1.

3. An oral care composition according to claim 2, which is tooth paste.

4. An oral care composition according to claim 2, which is mouth wash or liquefied tooth paste.

5. A food comprising the antimicrobial flavor according to claim 1.
